Europäisches Patentamt

European Patent Office

Office européen des brevets

(11) Numéro de publication: **0 197 810**
**A1**

# (12) DEMANDE DE BREVET EUROPEEN

(21) Numéro de dépôt: **86400477.5**

(22) Date de dépôt: **06.03.86**

(51) Int. Cl.4: **A61B 5/10**

(30) Priorité: **06.03.85 FR 8503296**

(43) Date de publication de la demande:
**15.10.86 Bulletin 86/42**

(84) Etats contractants désignés:
**AT BE CH DE FR GB IT LI SE**

(71) Demandeur: **Vanoni, Régis**
**11, Route de Saint Pancrace**
**F-06100 Nice(FR)**

(72) Inventeur: **Vanoni, Régis**
**11, Route de Saint Pancrace**
**F-06100 Nice(FR)**

(74) Mandataire: **Nony, Michel**
**Cabinet NONY & CIE 29, rue Cambacérès**
**F-75008 Paris(FR)**

(54) **Procédé et dispositif de reconnaissance d'un individu.**

(57) L'invention est relative à un procédé de reconnaissance d'un individu.

On émet un premier signal en direction d'une de ses artères, on relève le signal Doppler résultant à l'aide d'un capteur (1A), et on enregistre ce signal Doppler dans une mémoire (23) ; puis ultérieurement, on émet en direction de la même artère un deuxième signal sensiblement identique au premier signal, et on relève le signal Doppler résultant à l'aide d'un capteur (1), on compare à l'aide de moyens (22) la forme de ce deuxième signal Doppler à la forme du signal Doppler précédemment mis en mémoire, résultant de la première émission et on reconnaît l'individu si les formes des deux signaux se correspondent.

Fig. 2

EP 0 197 810 A1

## Procédé et dispositif de reconnaissance d'un individu.

La présente invention concerne un procédé et un dispositif de reconnaissance d'un individu.

Le problème de la reconnaisance des individus se pose de plus en plus souvent, et dans les domaines les plus variés.

C'est ainsi, par exemple, que le développement de l'informatique et de la télématique a entraîné la multiplication de terminaux, à partir desquels sont accessibles un grand nombre d'informations dont certaines sont confidentielles. Certains de ces terminaux sont en outre interactifs, c'est-à-dire que l'on peut, par leur intermédiaire, donner des ordres à un ordinateur ou modifier des données qu'il contient en mémoire.

Ce problème se rencontre en particulier dans le domaine bancaire où il est désormais possible, pour le titulaire d'un compte, d'effectuer certaines opérations à partir d'un terminal de type "Minitel", connectable sur n'importe quelle prise téléphonique.

La solution adoptée jusqu'à présent a consisté à exiger de l'utilisateur qu'il fournisse, préalablement à toute consultation ou opération, un "code confidential" qui, seul, permet l'accès à l'ordinateur, c'est-à-dire à un compte donné dans l'exemple ci-dessus.

Cette solution n'est toutefois pas entièrement satisfaisante, puisque l'accès est permis par la simple connaissance du code confidentiel même si ce code a été obtenu par hasard ou même par des moyens frauduleux.

Egalement dans le domaine bancaire, le problème de la reconnaissance des individus se pose lors de l'utilisation de cartes de crédit ou de cartes de mémoire dans les guichets automatiques. Là encore, tout détenteur d'une carte peut l'utiliser, sous réserve de connaître le code confidentiel. Ce problème est en fait très général, et on le rencontre également, par exemple, dans la technique des "badges" magnétiques ou des serrures électroniques.

On a donc recherché des moyens de reconnaître physiquement un individu, indépendamment de tout code ou de tout moyen qui lui soit extérieur.

C'est ainsi que l'on a proposé de traiter par ordinateur la voix ou la forme de la rétine ou les empreintes digitales du sujet, mais les résultats sont peu fiables et nécessitent en outre un traitement informatique très lourd.

La présente invention vise à fournir un procédé et un dispositif de reconnaissance d'un individu, qui soit facile à mettre en oeuvre, peu côuteux, et fiable.

A cet effet, l'invention a pour objet un procédé de reconnaissance d'un individu, caractérisé par le fait que l'on émet un premier signal en direction d'une de ses artères, que l'on relève le signal Doppler résultant, et que l'on enregistre ce signal Doppler dans une mémoire ; puis qu'ultérieurement, on émet en direction de la même artère un deuxième signal sensiblement identique au premier signal, que l'on relève le signal Doppler résultant, que l'on compare la forme de ce deuxième signal Doppler à la forme du signal Doppler précédemment mis en mémoire, résultant de la première émission, et que l'on reconnaît l'individu si les formes des deux signaux se correspondent.

La correspondance entre les formes des deux signaux peut être établie au moyen de tout critère prédéterminé.

Des moyens pour émettre et relever lesdits signaux Doppler sont déjà connus en eux-mêmes, et utilisés dans le domaine médical pour la mise en oeuvre des techniques de diagnostic par vélocimétrie sanguine.

Mais il a été constaté d'une façon surprenante, que, la forme du signal Doppler ainsi obtenu est une caractéristique propre à chaque individu comme, par exemple, ses empreintes digitales. Il en résulte qu'en enregistrant dans un premier temps le signal d'un individu, on peut ultérieurement reconnaître cet individu avec certitude.

Il est ainsi possible de savoir s'il peut avoir accès à telle information ou effectuer telle opération.

Les signaux utilisés peuvent être par exemple des signaux électromagnétiques, et il a été constaté que l'on obtenait de particulièrement bons résultats avec des signaux haute fréquence (HF) compris dans la bande des 9 à 11 MHz, de préférence entre 9,5 et 10,5 MHz.

L'artère choisie est de préférence, une artère digitale telle que l'artère d'un des deux index, de manière que la réalisation du test soit la plus simple possible. L'identification s'effectue alors en faisant simplement poser le doigt du sujet sur une sonde appropriée.

Le signal Doppler résultant de la première émission mentionnée ci-dessus, peut tout d'abord être enregistré dans la mémoire d'un ordinateur.

Ce sera le cas, en particulier, lorsque l'on souhaitera contrôler l'accès à cet ordinateur, par exemple dans les applications bancaires du "Minitel".

Mais ce signal Doppler peut également être enregistré sur une carte telle qu'une carte magnétique ou une carte à mémoire, ou tout autre support.

La carte bancaire à mémoire pourra, par exemple, comporter cet enregistrement, ce qui permettra de vérifier localement que son utilisateur en est bien le titulaire.

Le signal peut d'ailleurs être enregistré simultanément sur une carte et dans la mémoire d'un ordinateur, afin d'augmenter la fiabilité du test.

L'invention a également pour objet un dispositif pour la reconnaissance d'un individu, caractérisé par le fait qu'il comprend des moyens pour effectuer une mesure Doppler sur une artère d'un individu, des moyens de mémorisation contenant, sous forme codée, le signal résultant d'une telle mesure préalablement effectuée, et des moyens pour comparer la forme du signal de sortie des moyens de mesure, à la forme du signal contenu en mémoire.

Les moyens de mesure peuvent, dans un mode de réalisation particulier de l'invention, comprendre deux sondes électromagnétiques HF sensiblement identiques, et des moyens pour détecter les battements résultant du fonctionnement simultané des deux sondes.

Comme cela a été mentionné précédemment, les moyens de mémorisation peuvent comprendre la mémoire d'un ordinateur, ou une carte, telle qu'une carte magnétique ou une carte à mémoire, ou tout autre support.

Les moyens de comparaison comprendront de préférence un ordinateur programmé. Un programme de reconnaissance de forme relativement simple, tel qu'il en existe déjà, peut être utilisé à cet effet.

On décrira maintenant à titre d'exemple non limitatif un mode de réalisation particulier de l'invention en référence aux dessins schématiques annexés dans lesquels :

-La figure 1 est un schéma électrique d'un capteur permettant la mise en oeuvre du procédé selon l'invention et,

-la figure 2 est un diagramme d'un dispositif selon l'invention.

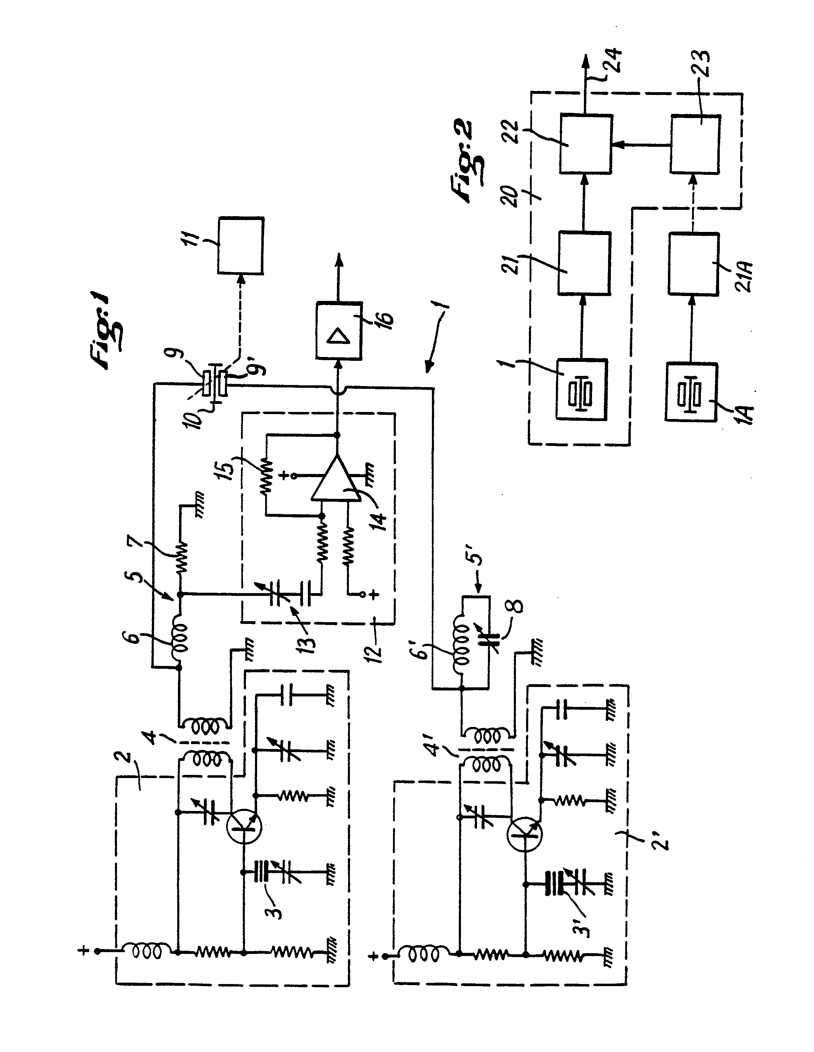

Le capteur 1 de la figure 1 comprend tout d'abord deux oscillateurs 2 et 2' pilotés chacun par un quartz 3 et 3' respectivement.

Les deux oscillateurs 2 et 2' sont le plus identique possible l'un à l'autre. En particulier les deux quartz 3 et 3' doivent avoir la même fréquence, de préférence aux environs de 10MHz, at la variation de cette fréquence, en fonction notamment du temps et de la température, doit être la même que pour les deux quartz.

L'accord des deux circuits oscillateurs 2 et 2' est obtenu par réglage de leurs condensateurs variables de la manière qui sera décrite ci-après.

La sortie de l'oscillateur 2 est transmise par l'intermédiare d'une self d'isolation 4 à un circuit oscillant 5 composé d'une self 6 et d'une résistance 7 de mise à la masse.

La sortie de l'oscillateur 2' est transmise par l'intermédiaire d'une self d'isolation 4' à un circuit oscillant 5' formé d'une self 6' et d'un condensateur variable 8.

Les deux signaux haute fréquence ainsi obtenus prélevés respectivement aux points communs des selfs 4 et 6 d'une part et 4' et 6' d'autre part, sont chacun transmis à une sonde 9 et 9' respectivement. Les deux sondes 9 et 9' sont, par exemple, chacune formée d'une masse en acier inoxydable, et sont séparées l'une de l'autre par une couche d'une résine époxy 10 assurant l'isolation.

Les deux sondes 9 et 9' et la résine 10 sont assemblées de manière à former un bouton susceptible de recevoir un des index de l'utilisateur.

A cet effet, la surface d'appui du bouton est constituée pour moitié par une surface de chacune des sondes 9 et 9', le plan de la couche d'époxy 10 étant perpendiculaire à cette surface.

De préférence, le bouton constitué par les sondes 9 et 9' et la résine 10 est un bouton poussoir, relié par l'intermédiaire d'un relai à un circuit de temporisation 11 permettant le fonctionnement du dispositif pendant un certain temps après que le bouton ait été enfoncé.

Du fait de l'écoulement sanguin dans l'artère digitale de l'utilisateur, les fréquences détectées au niveau des sondes 9 et 9' sont décalées par rapport à la fréquence des oscillateurs 2 et 2'. Il en résulte, par effet Doppler, un signal de battement qui est prélevé au point commun de la self 6 et de la résistance 7.

Ce signal, qui est un signal haute fréquence, est fourni à l'entrée d'une unité de détection et de préamplification 12, constituée pour l'essentiel de condensateurs 13 connectés en série entre l'entrée dans le circuit 12 et l'entrée dans un amplificateur opérationnel 14. Cet amplificateur est bouclé par une résistance de contre réaction 15, choisie de telle sorte que le gain de l'amplificateur 14 soit de l'ordre de 1000.

La sortie de l'amplificateur 14 est alors un signal basse fréquence, qui est amplifié au moyen d'un amplificateur basse fréquence traditionnel 16.

C'est le signal de sortie de l'amplificateur 16 qui est utilisé pour la reconnaissance de l'individu dont l'index est posé sur les sondes 9 et 9'.

L'accord des oscillateurs 2 et 2' est réalisé à l'aide des condensateurs variable de ces circuits, de façon à n'obtenir aucun battement à l'entrée du circuit 12 lorsque les sondes 9 et 9' sont libres.

On observe alors que, lorsqu'un utilisateur pose son index sur le bouton constitué par ces sondes, le signal basse fréquence obtenu a une fréquence d'environ 1Hz et une forme qui est caractéristique de l'individu.

Le circuit de temporisation 11 est réglé de manière à activer le dispositif pendant une durée de l'ordre de 3 à 5 secondes de manière à détecter trois périodes du signal basse fréquence, afin d'améliorer la reconnaissance.

La figure 2 représente un dispositif 20 selon l'invention dans lequel est intégré un capteur 1 tel que décrit ci-dessus.

La sortie du capteur 1 est appliquée, par l'intermédiaire d'un circuit d'interface 21, à une unité de calcul 22 telle que l'unité centrale d'un ordinateur. A cette unité de calcul 22, est connectée une mémoire 23 dans laquelle sont stockés les signaux caractéristiques des utilisateurs autorisés, soit de l'ordinateur comprenant l'unité 22, soit de tout organe relié à cet ordinateur. La sortie 24 de l'unité de calcul 22, peut donc soit être bouclée sur cet ordinateur lui-même, soit être connectée à tout périphérique en vue de la validation de ce dernier.

Par exemple, dans l'application envisagée ci-dessus du dispositif d'accès à une banque de données, le signal de sortie 24 de l'unité 22 permettra l'utilisation d'un terminal auquel est associé le capteur 1.

La comparaison entre le signal provenant du capteur et le ou les signaux stockés dans la mémoire 23 est effectuée dans l'unité 22 à l'aide d'un programme convenable de reconnaissane de forme.

Le chargement de la mémoire 23 s'effectue, lors de la délivrance à un utilisateur donné d'une autorisation d'utilisation, à l'aide d'un capteur 1A similaire au capteur 1, par l'intermédiaire d'un circuit d'interface 21A. Ainsi, lorsqu'un individu quelconque met son index sur le bouton du capteur 1, la temporisation 11 entraîne la détection du signal basse fréquence caractéristique et sa transmission à l'unité centrale 22. Celle-ci détermine alors si le signal reçu correspond à un des signaux contenus dans la mémoire 23.

L'émission du signal par le capteur 1 peut être combinée à l'émission par un terminal associé d'un numéro de code ou d'un numéro de compte, auquel cas, l'unité 22 détermine si le signal caractéristique reçu du capteur 1 correspond au signal associé à ce numéro de code ou ce numéro de compte dans la mémoire 23.

Diverses variantes et modifications peuvent bien entendu être apportées à la description ci-dessus sans sortir pour autant du cadre, ni de l'esprit de l'invention. En particulier l'invention n'est absolument pas limitée au domaine bancaire, l'unité de calcul pouvant être par exemple reliée à une serrure électronique ou à une base de données quelconque.

**Revendications**

1 -Procédé de reconnaissance d'un individu, caractérisé par le fait que l'on émet un premier signal en direction d'une de ses artères, que l'on relève le signal Doppler résultant, et que l'on enregistre ce signal Doppler dans une mémorie ; puis qu'ultérieurement, on émet en direction de la même artère un deuxième signal sensiblement identique au premier signal, que l'on relève le signal Doppler résultant, que l'on compare la forme de ce deuxième signal Doppler à la forme du signal Doppler précédemment mis en mémoire, résultant de la première émission et que l'on reconnaît l'individu si les formes des deux signaux se correspondent.

2 -Procédé selon la revendication 1, caractérisé par le fait que lesdits signaux sont des signaux électromagnétiques.

3 -Procédé selon la revendication 2, caractérisé par le fait que lesdits signaux électromagnatiques sont des signaux continus périodiques de fréquence comprise entre 9 et 11 MHz et de préférence entre 9,5 et 10,5 MHz.

4 -Procédé selon l'une quelconque des revendications 1 à 3, caractérisé par le fait que ladite artère est l'artère digitale d'un index.

5 -Procédé selon l'une quelconque des revendications 1 à 4, caractérisé par le fait que le signal Doppler résultant de la première émission est enregistré dans la mémoire d'un ordinateur.

6 -Procédé selon l'une quelconque des revendications 1 à 5, caractérisé par le fait que le signal Doppler résultant de la première émission est enregistré sur une carte telle qu'une carte magnétique ou une carte à mémoire.

7 -Procédé selon l'ensemble des revendications 5 et 6, caractérisé par le fait que l'on compare en outre le signal enregistré sur la carte, au signal enregistré dans la mémoire de l'ordinateur.

8 -Dispositif pour la reconnaissance d'un individu, caractérisé par le fait qu'il comprend des moyens - (1) pour effectuer une mesure Doppler sur une artère d'un individu, des moyens de mémorisation - (23) contenant sous forme codée le signal résultant d'une telle mesure préalablement effectuée, et des moyens (22) pour comparer la forme du signal de sortie des moyens de mesure, à la forme du signal contenu en mémoire.

9 -Dispositif selon la revendication 8, caractérisé par le fait que les moyens de mesure comprennent deux sondes électromagnétiques HF (9,9') sensiblement identiques et des moyens pour détecter les battements résultant du fonctionnement simultané des deux sondes.

10 -Dispositif selon l'une quelconque des revendications 8 et 9, caractérisé par le fait que les moyens de mémorisation comprennent la mémoire d'un ordinateur.

11 -Dispositif selon l'une quelconque des revendications 8 et 9, caractérisé par le fait que les moyens de mémorisation comprennent une carte telle qu'une carte magnétique ou une carte à mémoire.

12 -Dispositif selon l'une quelconque des revendications 8 à 11, caractérisé par le fait que les moyens de comparaison comprennent un ordinateur programmé.

Fig.1

Fig.2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 872 443  (OTT)<br>* Figures 1,2; colonne 1, ligne 55 - colonne 2, ligne 19; colonne 3, ligne 1 - colonne 6, ligne 9 * | 1,8 | A 61 B   5/10 |
| A | | 6,10-12 | |
| | --- | | |
| Y | IEEE SPECTRUM, vol. 21, no. 12, décembre 1984, pages 44-51, New York, US; P.N.T. WELLS: "Medical ultrasonics"<br>* Page 48, colonne de gauche, lignes 37-61 * | 1,8 | |
| | --- | | |
| A | US-A-4 048 986  (OTT)<br><br>* Figures 1-6; colonne 1, lignes 37-47; colonne 2, ligne 54 - colonne 6, ligne 21 * | 1,2,5, 7-10, 12 | **DOMAINES TECHNIQUES RECHERCHES (Int. Cl.4)**<br><br>A 61 B<br>G 07 C |
| | ----- | | |

Le présent rapport de recherche a été établi pour toutes les revendications

| Lieu de la recherche | Date d'achèvement de la recherche | Examinateur |
|---|---|---|
| LA HAYE | 16-06-1986 | CHEN A.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82